# NEUE EUROPÄISCHE PATENTSCHRIFT

(11) **EP 1 935 870 B2**
(45) Veröffentlichungstag und Bekanntmachung des Hinweises auf die Entscheidung über den Einspruch: **31.08.2016**
(45) Hinweis auf die Patenterteilung: 08.08.2012
(21) Anmeldenummer: 07023635.1
(22) Anmeldetag: 06.12.2007
(51) Int. Cl.: C07C 209/36, C07C 211/50

(54) **Verfahren zur Herstellung von Diaminotoluol durch katalytische Hydrierung von Dinitrotoluolen**
Process for preparing toluenediamines by catalytic hydrogenation
Procédé destiné à la fabrication de toluènediamine par hydrogénisation catalytique de dinitrotoluènes

(30) Priorität: 19.12.2006 DE 102006060572
(43) Veröffentlichungstag der Anmeldung: 25.06.2008
(73) Patentinhaber: Covestro Deutschland AG, 51373 Leverkusen (DE)
(72) Erfinder: Pohl, Fritz, Dr., 25541 Brunsbüttel (DE); Lorenz, Wolfgang, Dr., 41540 Dormagen (DE); Padeken, Lars, Dr., 40223 Düsseldorf (DE); Steffens, Friedhelm, 51373 Leverkusen (DE); Wiechers, Gerhard, Dr., 51381 Leverkusen (DE); Pennemann, Bernd, Dr., 51467 Bergisch Gladbach (DE)
(74) Vertreter: Levpat

(56) Entgegenhaltungen:
- EP-A- 0 019 454
- EP-A1- 0 757 034
- WO-A-02/062729
- CA-A1- 1 034 603
- US-A- 3 356 728

## Beschreibung

Die Erfindung betrifft ein Verfahren zur Herstellung von Toluylendiamin (TDA), bei dem Dinitrotoluol (DNT) mit Wasserstoff in Gegenwart eines Katalysators umgesetzt wird, gekennzeichnet, durch die Merkmale des Patentanspruchs 1.

Toluylendiamine sind Zwischenprodukte fürdie Herstellung von Toluylendiisocyanaten (TDI), die wichtige, im großtechnischen Maßstab hergestellte Vorprodukte zur Herstellung von Polyurethanen sind. Ihre Herstellung durch katalytische Hydrierung von Dinitrotoluolen ist bekannt und vielfach beschrieben (Ullmann's Enzyklopädie dertechnischen Chemie, 4.Auflage, Band 7, Seite 393 ff, 1973, Verlag Chemie Weinheim / New York). Die industrielle Herstellung der Toluylendiamine erfolgt vorwiegend durch Umsetzung eines durch Nitrierung von Toluol mit Salpetersäure zugänglichen Gemisches isomerer Dinitrotoluole. Handelsübliche Gemische isomerer Dinitrotoluole werden vorwiegend mit Salpetersäure in Gegenwart von Schwefelsäure als Katalysator in einem zweistufigen isothermen Nitrierprozess unter intermediärer Bildung der entsprechenden Mononitrotoluole als Roh-DNT hergestellt. Sie werden anschließend in der Reaktion nachgeschalteten Stufen, vorwiegend in Wäschen, aufbereitet und so weitgehend von gelösten Schwefel- und Salpetersäuregehalten wie auch von in den Reaktionsstufen gebildeten Nebenkomponenten, z.B. Kresolen und deren Abbauprodukten, befreit.

Typische handelsübliche DNT-Produkte weisen DNT-Gehalte > 98,5 Gew.-%, weniger als 0,1 Gew.-% Mononitrotoluol, weniger als 0,1 Gew.-%Trinitrotoluol und weniger als 0,1 Gew.-% sonstiger Nebenkomponenten sowie geringe Restmengen an Toluol, bezogen auf das Gewicht des DNT-Produkt-Gemisches, bei DNT-Ausbeuten von > 98 % sowie Toluolumsätzen von > 99,9 % auf. Wesentlich ist ebenfalls das Gewichts-Verhältnis der 2,4- und 2,6-DNT-Isomeren in der Summe zu den 2,3-, 3,4-, 2,5 und 3,5-DNT-Isomeren in der Summe. Handelsüblich spezifikationsgerecht liegt der Gehalt der 2,4- und 2,6- DNT-Isomeren in der Summe im Roh-DNT bei > 95 Gew.-%, bezogen auf das Gewicht des Roh-DNT. Bevorzugt beträgt dabei der Gehalt des 2,4-DNT 79,0 - 81,0 Gew.-%, bezogen auf die Summe der Gewichte des 2,4-DNT und des 2,6-DNT. Entsprechend beträgt der Gehalt des 2,6-DNT 19,0 - 21,0 Gew.%, bezogen auf die Summe der Gewichte des.2;4-DNT und des 2,6-DNT.

Die katalytische Hydrierung dieser handelsüblichen DNT-Produkte kann unter Mitverwendung eines inerten Lösungsmittels oder in Substanz erfolgen, wobei die Gemische dann vor Durchführung der Hydrierung auf-geschmolzen werden. Sie kann sowohl diskontinuierlich als auch kontinuierlich unter Verwendung der üblichen Reaktoren durchgeführt werden. Für den wirtschaftlichen Erfolg des angewandten Verfahrens wesentlich sind neben einer kontinuierlichen Reaktionsführung vor allem die mit dem angewandten Verfahren erzielbaren Selektivitäten der Umsetzung sowie die Kapazitäten und Standzeiten der eingesetzten Katalysatoren.

US-B-3 356 728 offenbart ein verbessertes kontinuierliches Verfahren zur Herstellung von aromatischen Aminen durch katalytische Hydrierung aromatischer Polynitroaromaten in einem Schlammphasenreaktor, wobei das Verfahren beispielhaft an der Umsetzung von Dinitrotoluol erläutert wird. Nach der Lehre von US-B-3 356 728 erfolgt die katalytische Hydrierung von Dinitrotoluol in diesem Reaktionssystem im Hinblick auf Selektivität, Katalysatorstandzeit und Durchsatz besonders effektiv, wenn
■ die Reaktionszone bei der Umsetzung stets mit Wasserstoff gesättigt ist,
■ die aromatische Polynitroverbindung dem System unter Einhaltung eines bestimmten Gewichtsverhältnisses zu dem im Reaktionssystem vorhandenen Katalysator zugesetzt wird ("Katalysatorbeladung") und
■ die Konzentration der zugesetzten aromatischen Polynitroverbindung in der Reaktionszone einen angegebenen Grenzwert nicht übersteigt.

US-B-3356 728 beansprucht für die sogenannte Katalysatorbeladung ("Verhältnis der zugesetzten Menge an aromatischer Polynitroverbindung in kg-Äquivalenten an Nitrogruppen per Stünde zu dem im Reaktor vorhandenen Katalysator in kg") den Arbeitsbereich < 0.15, bevorzugt den Arbeitsbereich von 0,01 bis 0,11, offenbart als einzuhaltende max. Konzentration der aromatischen Nitroverbindung im Reaktionsgemisch 0,1 Gew.%, bevorzugt kleiner als 0,015 Gew.%, bezogen auf das Gewicht des Reaktionsgemischs.

Nach der Lehre von US-B- 3 356 728 führen die beanspruchten Katalysatorbeladungen zu hohen Konzentrationen an aktivem Katalysator im Reaktionssystem, so dass die eingespeiste aromatische Polynitroverbindung nach ihrem Eintritt in das Gemisch sofort zu dem gewünschten Amin umgesetzt wird, dadurch die Konzentration an unreduzierter Nitroverbindung in dem Reaktionssystem zu jeder Zeit kleiner 0,005 Gew% gehalten wird. Nach der Lehre von US-B- 3 356 728 wird durch diese geringe Konzentration eine schnelle Vergiftung des Katalysators verhindert, werden darüber hinaus bei der Umsetzung der aromatischen Polynitroverbindung höhere Ausbeuten und eine verbesserte Produktreinheit bei wesentlich reduzierten Kosten erhalten.

Die Vermeidung unzulässig hoher Konzentrationen an nicht reduzierter Nitroverbindung im Reaktionsgemisch katalytischer Hydrierungen aromatischer Polynitroverbindungen ist ebenfalls Gegenstand von US-B-3 499 034. US-B-3499034 offen bart als einzuhaltende maximale Konzentration an nicht reduzierter aromatischer Nitroverbindungen 0,5 Gew-%, bezogen auf das Reaktionsgemisch. Nach der Lehre von US-B-3 499 034 bewirken diese geringen Konzentrationen an nicht reduzierter Nitroverbindung vor allem niedrige Konzentrationen an den bekanntermaßen bei der katalytischen Hydrierung von Nitroverbindungen auch gebildeten Azoxy-, Azo- und Hydrazoverbindungen, die nach der Lehre von US-B-3 499 034 selbst Teer-artige Verbindungen darstellen, zwar reduziert werden können, jedoch mit Schwierigkeiten und allein unter deutlicher Verlangsamung der angestrebten katalytischen Hydrierung der aromatischen Polynitroverbindung.

Nach der Lehre von EP 0 171 052 B1 ist die Bildung der teerartigen Zwischenprodukte bei der katalytischen Hydrierung von aromatischen Nitroverbindungen nicht nur von der Konzentration an unreduzierter Nitroverbindung sondern ebenfalls von der Nitroverbindung selbst abhängig. Nach der Lehre von EP 0171 052 B1 verläuft die katalytische Hydrierung aromatischer Nitroverbindungen besonders gut, wenn als aromatische Nitroverbindungen Gemische von wenigstens 25 Gew% Mononitro-nonamino-Aromaten mit wenigstens 25 Gew% an Dinitro- oder Mononitro-amino-Aromaten eingesetzt werden. Der Vorteil der offenbarten Reaktionsführung ist angesichts des nachfolgend notwendigen Aufwands zur destillativen Trennung der Hydrierprodukte jedoch begrenzt, so dass im großtechnischen Maßstab die katalytische Hydrierung aromatischer Polynitroverbindungen üblicherweise unter Beachtung der beispielhaft durch US-B-3 356 728 und US-B-3 499 034 skizzierten Prinzipien erfolgt.

Nach der Lehre von GB-B-832 153 können neben der unreduzierten aromatischen Nitroverbindung und deren intermediären Azoxy-, Azo- und Hydrazoverbindungen ebenfalls von der zu hydrierenden Nitroverbindung beinhaltete Verunreinigungen zu einer starken Beeinflussung der angestrebten katalytischen Hydrierung der Nitroverbindung führen. Nach der Lehre von GB-B-832 153 sind Nitrophenole und Nitrocresole, üblicherweise in geringen Mengen in handelsüblichen Dinitrotoluol-Isomerengemischen vorhanden, Zersetzungsbeschleunigersowie starke Katalysatorgifte, so dass deren Konzentration als kritisch im Hinblick auf die Verfahrenssicherheit wie auch im Hinblick auf die Effizienz der angestrebten katalytischen Hydrierung der Nitroverbindung zum entsprechenden Amin anzusehen ist. Nach der Lehre von GB-B-832 153 sollte die in die katalytische Hydrierung eingesetzte Nitroverbindung weniger als 500 ppm "Nitrophenole", bevorzugt weniger als 20 ppm "Nitrophenole" enthalten, wobei nach der Lehre von GB-B-832 153 unter "Nitrophenole" die Summe nitrophenol- und nitrokresolartiger Verbindungen zu verstehen ist.

Der Einfluss nitrophenolartiger Verunreinigungen wird ebenfalls in EP 0 019 454 B1 behandelt. Nach der Lehre von EP 0 019 454 B1 ist die Entfernung der nitrophenolartigen Verunreinigungen weitgehend unnötig, ist es zur Vermeidung einer Katalysatorvergiftung sowie der Zersetzung des gebildeten Diamins jedoch wesentlich, bei derkatalytischen HydrierungtechnischerDinitrotoluoe deren Säuregehalt, ausgedrückt als HNO3, unter 6 000 ppm, bezogen auf das Gewicht des Dinitro-toluols, zu senken. EP 0 019 454 B1 offenbart ein Verfahren, bei dem das rohe Dinitrotoluol allein mit Wasser gewaschen wird, auf den Gebrauch wässriger alkalischer Lösungen zur Abtrennung von nitrophenolartigen Verunreinigungen wird verzichtet.

Differenzierter sind die Aussagen von US-B-4 482 769. Nach der Lehre von US-B-4 482 769 ist die Wäsche der technischen Dinitrotoluolgemische mit wässriger alkalischen Lösungen vorteilhaft, sollte die Wäsche jedoch so erfolgen, dass die wässrige Phase einen pH-Wert im Bereich von 5,8 bis 6,4 aufweist. Nach der Lehre von US-B-4 482 769 führt ein solcher pH-Wert der Wäsche dazu, dass weitgehend alle sauren Komponenten aus dem Dinitrotoluol entfernt werden, allein das biologisch schlecht abbaubare 2,4-Dinitroorthokresol als Nebenkomponente im Dinitrotoluol verbleibt. Nach der Lehre von US-B-4 482 769 wird mit dem beanspruchten Verfahren zum einen der Einfluss saurer Komponenten vorteilhaft verhindert, beeinflusstzum anderen ein geringer Gehalt an 2,4-Dinitroorthokresol nachfolgende Hydrierungen des so aufbereiteten Dinitrotoluols nicht.

Überraschenderweise wurde nun gefunden, dass bei der Herstellung von Toluylendiamin, bei der Dinitrotoluol mit Wasserstoff in Gegenwart eines Katalysators umgesetzt wird, neben den nach dem Stand der Technik bekannten Parametern auch der Kohlendioxidgehalt des in der Umsetzung eingesetzten Dinitrotoluols einen wesentlichen Einfluss auf die angestrebte Umsetzung hat, bei geringen Kohlendioxidgehalten des Dinitrotoluols wesentlich bessere Katalysatorstandzeiten bei erhöhter Selektivität der Umsetzung erzielt werden.

Gegenstand der Erfindung ist daher ein Verfahren zur Herstellung von Toluylendiamin, bei dem Dinitrotoluol mit Wasserstoff in Gegenwart eines Katalysators umgesetzt wird, gekennzeichnet, durch die Merkmale des Patentanspruchs 1.

Eine geeignete Analysenmethode zur Bestimmung des Gehalts an Kohlendioxid in physikalisch gelöster oder chemisch gebundener Form, die im Rahmen der Durchführung des erfindungsgemäßen Verfahrens eingesetzt werden kann, ist im Abschnitt "Beispiele" unter dem Titel "Beschrelbung der Analysenmethode zur quantitativen Bestimmung von Kohlendioxid in physikalisch gelöster oder chemisch gebundener Form in Dinitrotoluol" offenbart. Dabei ist im Rahmen dieser Erfindung unter dem Gehalt an Kohlendioxid in physikalisch gelöster oder chemisch gebundener Form der Gesamtgehalt an Kohlendioxid, welcher in physikalisch gelöster und / oder chemisch gebundener Form vorliegen kann, zu verstehen.

Das erfindungsgemäße Verfahren ist dadurch gekennzeichnet, dass
a) Toluol mit Nitriersäure zu Mononitrotoluol umgesetzt wird, wobei ein Mononitrotoluole enthaltenes Reaktionsgemisch erhalten wird, und
b) das Mononitrotoluole enthaltende Reaktionsgemisch in eine Mononitrotoluole enthaltende organische Phase und eine Schwefelsäure enthaltende wässrige Phase aufgetrennt wird, und
c) die Mononitrotoluole enthaltende organische Phase mit Nitriersäure umgesetzt wird,
   wobei ein Dinitrotoluole (Dinitrotoluol-Isomerengemisch) enthaltendes Reaktionsgemisch erhalten wird, und
d) das Dinitrotoluole enthaltende Reaktionsgemisch in eine Dinitrotoluole enthaltende organische Phase und eine Schwefelsäure enthaltende wässrige Phase aufgetrennt wird, und
e) die Dinitrotoluole enthaltende organische Phase mit Wasser in einer mehrstufigen Extraktion umfassend Vermischung und Phasentrennung in jeder Stufe gereinigt wird, und
f) die so aufbereiteten Dinitrotoluole enthaltend 2,4-DNT in Gehalten von 74- 81 Gew%, 2,6-DNT in Gehalten von 17 - 21 Gew.%, sowie 2,3-DNT, 2,5-DNT, 3,4-DNT und 3,5-DNT in Summe in Gehalten < 5,5 Gew.-%, jeweils bezogen auf das Gewicht der eingesetzten Dinitrotoluole, mit Wasserstoff in Gegenwart eines Katalysators zu Toluylendiaminen umgesetzt wird,
   wobei
g) die Verweilzeit der Vermischung jeder Stufe der Extraktion in Schritt e) mindestens 4 Minuten und höchstens 60 Minuten beträgt, und
h) zumindest in der letzten Stufe der Extraktion in Schritt e) in die Vermischung zusätzlich ein Inertgas in einem solchen Gewichts-Verhältnis von Inertgas zu den Dinitrotoluole eingetragen wird, dass die erhaltenen, gereinigten Dinitrotoluole einen Gehalt an Kohlendioxid in physikalisch gelöster oder chemisch gebundener Form in der Summe von weniger als 0,175 mol-%, bevorzugt von weniger als 0,125 mol-%, ganz besonders bevorzugt von weniger als 0,075 mol-%, bezogen auf die molare Menge der Dinitrotoluole, aufweist.

In dem erfindungsgemäßen Verfahren kann die Reaktionsführung bei der Herstellung der Mononitrotoluole in Schritt a) sowie der Di-nitrotoluole in Schritt c) adiabat oder isotherm erfolgen. Bevorzugt erfolgt sie isotherm unter Einsatz von kontinuierlich durchströmten gekühlten Rührkesselkaskaden oder in Reihe geschalteten gekühlten Schleifenreaktoren, wie sie z.B. in EP 0 903 336 B1 bzw. DE10 2004 005 913 A1 beschrieben sind. Bei der Phasentrennung in den Schritten b) und d) können statische und dynamische Separatoren Verwendung finden. Bevorzugt werden statische Separatoren, wie sie z.B. in EP 0 903 336 B1 beschrieben sind, eingesetzt.

Bei dem erfindungsgemäßen Verfahren kann in Schritt e) die mehrstufige Extraktion der in Schritt d) erhaltenen organischen Phase unter Rückgewinnung der Hauptmenge derin derorganischen Phase enthaltenen Salpetersäure und / oder Schwefelsäure erfolgen. Dabei kann die Rückgewinnung ein- odermehrstufig unter Einsatz von kleinen Mengen unbeladenen Wassers in die einzelnen Stufen oder mehrstufig im Gegenstrom unter Einsatz größerer, auf jeder Stufe im Kreis geführter Wassermengen erfolgen. Geeignete Verfahren sind z.B. In EP 0 279 312 B1 bzw. EP 0 736 514 B1 beschrieben.

Bevorzugt erfolgt keine Säurenrückgewinnung. Dann wird bei dem erfindungsgemäßen Verfahren in Schritt e) die in Schritt d) erhaltene Dinitrotoluol enthaltende organische Phase mit Wasser in einer mehrstufigen Extraktion umfassend Vermischung und Phasentrennung in jeder Stufe gewaschen. In einer besonders bevorzugten Ausführungsform kann das zur mehrstufigen Extraktion der Dinitrotoluole (DinitrotoluolIsomerengemisch) eingesetzte Wasser in den verschiedenen Stufen unterschiedliche pH-Werte aufweisen. Bevorzugt wird dann in Schritt e) zur Extraktion der Dinitrotoluole in mindestens einer Stufe alkalisches Wasserund in mindestens einer Stufe neutrales Wasser eingesetzt. Bei einer mehr als zwei Stufen umfassenden Extraktion wird bevorzugt in mindestens einer Stufe alkalisches Wasser, in mindestens einer Stufe saures Wasser und in mindestens einer Stufe neutrales Wasser eingesetzt. Bei dieser bevorzugten Ausführungsform erfolgt die Extraktion in den Stufen als "Flüssig/Flüssig"-Extraktion. Dies wird durch eine geeignete Wahl der Temperaturen der eingesetzten Dinitrotoluole wie auch der als Extraktionsmittel eingesetzten wässrigen Phasen sichergestellt.

In einer weiteren bevorzugten Ausführungsform erfolgt die mehrstufige Extraktion zumindest auf einer Stufe unter Einsatz eines Apparates zum Mischen und Trennen praktisch nicht Ineinander lösbarer Flüssigkeiten unterschiedlichen spezifischen Gewichtes, wie er z.B. in DE-B- 1 135 425 beschrieben ist.

Kennzeichen des in DE-B-1 135 425 beschrieben Apparates sind eine als Extraktiöns- bzw. Waschkolonne ausgebildete Mischzone mit einem konzentrisch um die Mischzone angeordnetem Raum zur Phasentrennung, in den das die Mischzone verlassende Gemisch über ein Überlaufwehr mit umgebenden Hohlmantel der "abgeschnittenen" Waschkolonne eintritt und nach Maßgabe der Dichten in zwei Phasen aufgetrennt wird. Um den in DE-B-1 135 425 in Spalte 2 / Zeilen 35-52 und Spalte 3/Zeilen 1-12 in seinem Aufbau sowie in Spalte 3/Zeilen 29-47 in seiner Funktion beschrieben Apparat in dem erfindungsgemäßen Verfahren einzusetzen, sollte in dem Apparat zusätzlich noch eine Möglichkeit für die Zufuhrvon Inertgas in die Mischzone, beispielsweise durch eine zusätzliche Öffnung im Bereich des Bodens dieses Apparateabschnitts, eingerichtet werden.

Es können für die Extraktion in Schritt e) aber grundsätzlich jede Form von mehrstufigen Extraktionsverfahren und extraktionsapparaten umfassend Vermischung und Phasentrennung in jeder Stufe eingesetzt werden, vorausgesetzt dass die Verweilzeit der VermischungjederStufe der Extraktion in Schritte) mindestens 4 Minuten und höchstens 60 Minuten beträgt (Schritt g)), und zumindest in der letzten Stufe der Extraktion in Schritt e) in die Vermischung (d.h. in das Volumen, in dem die Vermissung stattfindet) zusätzlich ein Inertgas in einem solchen Gewichts-Verhältnis von Inertgas zu den Dinitrotoluolen eingetragen wird, dass die erhaltenen gereinigten Dinitrotoluole einen Gehalt an Kohlendioxid in physikalisch gelöster oder chemisch gebundener Form in der Summe von weniger als 0,175 mol.-%, bezogen auf die molare Menge der Dinitrotoluole, aufweist (Schritt h)).

Dabei ist die erforderliche Menge bzw. das erforderliche Gewichtsverhältnis von Inertgas zu den Dinitrotoluolen für den Fachmann leicht experimentell zu bestimmen, indem Versuche mit steigenden Mengen bzw. Gewichtsverhältnissen von Inertgas zu den Dinitrotoluolen durchgeführt werden, bis der geforderte Gehalt an Kohlendioxid in physikalisch gelöster oder chemisch gebundener Form erreicht ist.

Die nach der mehrstufigen Extraktion in Schritt e) erzielte Kohlendioxid-Beladung in physikalisch gelöster oder chemisch gebundener Form der DinitrotoluolIsomerengemische kann bevorzugt gaschromatisch per "Headspace GC" verfolgt werden. Eine geeignete Analysenmethode zur Bestimmung des Gehalts an Kohlendioxid in physikalisch gelöster oder chemisch gebundener Form, die bevorzugt im Rahmen der Durchführung des erfindungsgemäßen Verfahrens eingesetzt werden kann, ist im Abschnitt "Beschreibung der Analysenmethode zur quantitativen Bestimmung von Kohlendioxid in physikalisch gelöster oder chemisch gebundener Form in Dinitrotoluol" offenbart.

Die so aufbereiteten Dinitrotoluole (Dinitrotoluot-Isomerengemisch) werden bevorzugt in einer Vorlage gesammelt und aus dieser in flüssiger Form der bevorzugt kontinuierlich betriebenen Hydrierung zugeführt. Die der Vorlage bevorzugt kontinuierlich zufließenden Dinitrotoluole können sowohl vor der Sammlung in der Vorlage, in der Vorlage oder nach Entnahme aus der Vorlage einer weiteren Strippgasbehandlung oder einer andersartigen Kohlendioxidentfemung unterzogen werden. Wesentlich für das erfindungsgemäße Verfahren ist allein, dass das in die katalytische Hydrierung zur Herstellung von Toluylendiamin eingesetzte Dinitrotoluol einen Gehalt an Kohlendioxid in physikalisch gelöster oder chemisch gebundener Form von weniger als 0,175 mol-%, bezogen auf die molare Menge des eingesetzten Dinitrotoluols, aufweist.

Die katalytische Hydrierung des so aufbereiteten Dinitrotoluols kann unter Mitverwendung eines inerten Lösungsmittels oderin Substanz erfolgen. Sie erfolgt bevorzugt in Substanz unter Einsatz einer wässrigen Katalysatorsuspension. Sie kann sowohl diskontinuierlich als auch kontinuierlich unter Verwendung der üblichen Reaktoren durchgeführt werden. Beispiele hierfür sind Rührkessel, Blasensäulen, oder Schlaufenreaktoren, wie Loop-Venturi-Reaktoren, oder Strahlschlaufenreaktoren mit innerem und äußerem Kreislauf.

In einer bevorzugten Ausführung des erfindungsgemäßen Verfahrens kommt ein Strahlschlaufenreaktor mit innerem und äußerem Kreis lauf zum Einsatz, wie erbeispielsweise in EP 1 137623 B1 beschrieben ist.

In einer weiteren bevorzugten Form des erfindungsgemäßen Verfahrens erfolgt die katalytische Hydrierung des Dinitrotoluols mit einem Gehalt an Kohlendioxid in physikalisch gelöster oder chemisch gebundener Form von weniger als 0,175 mol-%, bezogen auf die molare Menge des eingesetzten Dinitrotoluols, in einem Schlammphasenreaktor mit integriertem Wärmeaustauscher, wie er beispielsweise in WO-A-96/11052 beschrieben ist. Dieser Reaktor weist als Wärmeaustauscher einen von der Reaktionsmasse im Reaktor unter- und überdeckten Ringraum auf, wobei der Ringraum eine Vielzahl vertikaler Durchtrittskanäle für die Reaktionsmasse aufweist und das Kühlmittel den Ringraum zwischen den Durchtrittskanälen für die Reaktionsmasse durchströmt. Wie in WO-A-96/11052 offenbart, ist dieser Reaktor in besonderer Weise geeignet, die bei der katalytischen Hydrierung der Dinitrotoluolgemische freiwerdende Reaktionswärme in Form nutzbaren Wasserdampfes abzuführen. Bei der katalytischen Hydrierung des erfindungsgemäßen Dinitrotoluols wird im Reaktor eine Betriebstemperaturvon 80 bis 200°C, bevorzugt von 100 bis 180°C und ein Druck von 5 bis 100 bar, bevorzugt 10 bis 50 bar, aufrechterhalten.

Die Wasserstoffzufuhr in das System erfolgt dabei bevorzugt so, dass stets der stöchiometrische Bedarf an Wasserstoff für die Umsetzung der eingespeisten Nitrogruppenäquivalente zu den entsprechenden Aminverbindungen gedeckt wird und zusätzlich der Reaktorinhalt unter besonderer Berücksichtigung der Oberflächen des eingesetzten Katalysators stets mit Wasserstoff gesättigt ist. Bevorzugt wird dies dadurch erreicht, dass der bevorzugt eingesetzte Schlammphasenreaktor über einen Begasungsrührer verfügt, mit Hilfe dessen eine weitgehend homogene, feine Verteilung des im Reaktionsgemischs suspendierten Katalysators wie auch des Wasserstoffs in Form von fein dispergierten Wasserstoffbläschen in dem Reaktionsgemisch erzeugt wird.

Nach der Lehre von US 2004/0073066 A1 ist es dabei vorteilhaft, durch Zusatz von unter den Hydrierbedingungen gasförmigen Inerten bzw. durch Einstellung eines entsprechenden Purgegasstromes zur Ausschleusung der mit dem Wasserstoff in den Reaktor eingetragenen gasförmigen Verunreinigungen die Reinheit des im Reaktor vorhandenen Wasserstoff auf 50 bis 97 Vol.%, bevorzugt von 70 bis 97 Vol.-%, ganz besonders bevorzugt auf 80 bis 95 Vol-%, einzustellen. Nach der Lehre von US 2004/007366 A1 zeigt sehr reiner Wasserstoff eine große Koaleszenzneigung im Reaktionssystem, führt diese dazu, dass unmittelbar nach der Dispersionszone sich die feinen Wasserstoffbläschen zu großen, in Summe eine geringe Oberfläche aufweisenden, Blasen vereinen. Bei geringeren Wasserstoffkonzentrationen tritt nach der Lehre von US 2004/007366 Aldiese Koaleszenz nicht auf, wird der Vorteil einer großen Oberfläche bei zu geringen Wasserstoffkonzentrationen durch einen zu geringen Stoffübergang aufgehoben.

In einer besonders bevorzugten Form des Verfahrens kommt ein Schlammphasenreaktor zum Einsatz, dessen Begasungsrührer zusätzlich als Axialförderer, insbesondere von Gas/Flüssigkeitsdispersionen, wie beispielsweise in EP 0 856 665 B1 beschrieben ausgebildet ist. Durch die besondere Wahl dieses Mischorgans wird zum einen eine sehr hohe Umwälzleistung der flüssigen Phase, zum anderen auf einfache Weise die angestrebte Verteilung des Wasserstoffs in Form fein dispergierter Wasserstoffbläschen in dem Reaktionsgemisch erreicht.

Wie oben ausgeführt, sind örtliche Überkonzentrationen bei der Dosierung der Dinitrotoluole in den Reaktor der katalytischen Hydrierung zu vermeiden. Die Dosierung kann dabei über Lanzen, Düsen oder mechanisch angetriebenen Mischeinrichtungen erfolgen. In einer bevorzugten Form erfolgt sie über eine Rührvorrichtung, wie sie beispielsweise in EP 1 637 220 A1 beschrieben ist. Diese Rührvorrichtung besteht wenigstens aus einem Begasungsrührer und einem Flügelmischer oder zwei Flüssigmischern, welche auf einer Welle angeordnetsind und jeweils eine Zuführung und wenigstens eine Austrittsöffnung aufweisen, wobei die Austrittsöffnungen des Begasungsrührers und des Flüssigkeitsmischers oder der Flüssigkeitsmischer in einem definierten Abstand zueinander stehen und das Verhältnis a/d vom Abstand a der Austrittsöffnungen zum Durchmesser d des Begasungsrührers oder Flüssigmischers 0,025 bis 0,5, vorzugsweise 0,05 bis 0,3, und das Verhältnis b/d vom Abstand b der Außenkante zum Durchmesser d des Begasungsrührers oder Flüssigmischers 0,01 bis 0,4, vorzugsweise 0,02 bis 0,2 beträgt. Die skizzierte Rührvorrichtung ist in besonderer Weise zur gleichzeitigen Einmischung von Gas- und Flüssigphasen in ein Reaktionsgemisch geeignet, wobei die optimierte Einmischung der Phasen im besonderen Maß örtliche Überkonzentrationen an eingetragenen Nitrogruppenäqiuvalenten vermeidet.

Als Katalysatoren können alle zur katalytischen Hydrierung von aromatischen Nitroverbindungen bekannten Hydrierkatalysatoren eingesetzt werden. Gut geeignet sind insbesondere die Metalle der 8. Nebengruppe des Periodensystems der Elemente oder Mischungen derselben, die beispielsweise auf Trägermaterialien wie Kohlenstoff oder Oxiden von Magnesium, Aluminium und/oder Silizium aufgebracht sein können. Vorzugsweise werden Raney-Eisen, -Kobalt und/oder -Nickel, insbesondere Nickel-haltige Katalysatoren wie beispielsweise Raney-Nickel-Katalysatoren, sowie Palladium- oder Platin-haltige Katalysatoren auf Trägermaterialien eingesetzt, deren Herstellung und Anwendung als Hydrierkatalysatorvon aromatischen Nitroverbindungen wie z.B. Nitrobenzol, Nitrotoluolen, Dinitrotoluolen, chlorierten Nitroaromaten und anderen bekannt ist und bereits öfters beschrieben wurde (EP 0 223 035 B1, EP 066 111 B1, EP-A-1 512 459).

In einer ganz besonders bevorzugt Ausführung des erfindungsgemäßen Verfahrens werden als Katalysatoren Raney-Nickel-Katalysatoren eingesetzt, wie sie in EP-A-1 512 459 beschrieben sind und zu deren Herstellung
1) die Schmelze einer Legierung aus 50 bis 95 Gew.-% Aluminium, 10 bis 50 Gew.-% Nickel, 0 bis 20 Gew.-% Eisen, 0 bis 15 Gew.% Cer, Cer-Mischmetall, Vanadium, Niob, Tantal, Chrom, Molybdän oder Mangan sowie gegebenenfalls auch weiteren glasbildenden Elementen mit einer Abkühlrate > 104 K/s durch Ausdrücken der Legierungsschmelze auf ein rotierendes Kühlrad oder in den Spalt zwischen zwei im Gegensinn rotierenden Kühlrädern oder durch Schrmelzextraktion abgekühlt wird, und
2) anschließend die rasch erstarrte Legierung einer Behandlung mit organischen oder anorganischen Basen unterzogen wird.

Diese Katalysatoren zeichnen sich im Vergleich zu herkömmlichen Raney-Nickel-Katalysatoren durch eine deutlich erhöhte Produkt-Selektivität und Katalysatorstandzeit insbesondere bei Reaktionstemperaturen > 120 °C. Durch ihren Einsatz kann mithin die bei der Hydrierung von Dinitrotoluolen freiwerden Reaktionswärmen besonders vorteilhaft zur Erzeugung von als Heizmedium einsetzbarem Wasserdampf genutzt werden.

Dem bevorzugt kontinuierlich betriebenen Reaktionssystem zur katalytischen Hydrierung der Dinitrotoluole wird das Reaktionsgemisch nach Maßgabe der Speisungen entnommen, bevorzugt kontinuierlich unter Rückhaltung des Katalysators im System. Besonders bevorzugt erfolgt die Entnahme unter Einsatz einer Querstromfiltration, wie sie z.B. in EP 0 634 391 B1 im Prinzip oderin EP 1 137 623 B1 in einer speziellen Ausführungsform beschrieben sind. Bei diesem Produktaustrag wird dem Reaktor ein Teilstrom entnommen, dieser über ein Querstromfilter geführt, dort dem Produktstrom ein Teilmenge unter Rückhaltung des Katalysators entnommen, abschließend der verminderte, bezüglich seines Katalysatorgehalts "aufkonzentrierte" Teilstrom wieder dem Reaktor zugeführt.

Das filtrierte Produkt weist eine hohe Reinheit auf und kann ohne weitere chemische Nachbehandlung nach dem Stand der Technik zu dem Endprodukt Toylendiamin aufbereitet werden.

Insgesamt zeichnet sich das erfindungsgemäße Verfahren zur Herstellung von Toluylendiamin, bei dem Dinitrotoluol mit Wasserstoff in Gegenwart eines Katalysators umgesetzt wird, durch eine stark verbesserte Wirtschaftlichkeit gegenüber dem Stand der Technik aus. Durch den Einsatz von Dinitrotoluol mit einem Gehalt an Kohlendioxid in physikalisch gelöster oder chemisch gebundener Form von weniger als 0,175 mol-%, bezogen auf die molare Menge des eingesetzten Dinitrotoluols, werden erhöhte Katalysatorselektivitäten und wesentlich verbesserte Katalysatorstandzeiten erzielt.

Nachfolgend wird das erfindungsgemäße Verfahren anhand bevorzugter Ausführungsformen näher erläutert.

### Beispiele:

### Beschreibung der Analysenmethode zur quantitativen Bestimmung von Kohlendioxid in physikalisch gelöster oder chemisch gebundener Form in Dinitrotoluol

Zur quantitativen Bestimmung von physikalisch gelöstem oder chemisch gebundenem Kohlendioxid in Dinitrotoluol wird eine definierte DNT-Probenmenge in einem durch ein Septum gasdicht verschließbarem Rollrandgläschen vorgelegt, eine definierte Menge an Schwefelsäure zugegeben, dann das im Gasraum vorhandene Kohlendioxid mittels "Headspace"-Gaschromatographie ("Dampfraumanalyse mittels Gaschromatographie") mit Wärmeleitfähigkeitsdetektion bei Kalibrierung mit externem Standard bestimmt.

### Geräte:

Gaschromatograph HP 5890 mit Wärmeleitfähigkeitsdetektor (Fa. Hewlett-Packard)
Headspace Sampler HP 19395A (Fa. Hewlett-Packard)
Trennbedingungen:
   stationäre Phase: Silicagel #12, gepackte Säule (10ft x 1/8 in)
   Trägergas: Helium (Fluß von 12,5 ml/min)
   Säulenofen, Temperatur: 200°C
   Einspritzblock, Temperatur: 200 °C
   Detektor, Temperatur: 250 °C
   Headspace-Zelle: 45 °C
   Inkubationszeit: 3,0 min
   Injektionsvolumen: 1 ml

### Durchführung der Analyse:

Für die Analyse werden 1,0 g der DNT-Probe auf +/- 0,1 mg in das Rollrandgläschen eingewogen. Nach Verschließen des Gläschens werden 0,50 ml Schwefelsäure (w = 0,330 g/kg) mittels einer Spritze durch das Septum injiziert, wird der Inhalt des Röllrandgläschens durch leichtes Schütteln gemischt, dann das Rollrandgläschen in den Headspace-Sampler gestellt und nach Temperierung mittels Head-space-GC analysiert.

Die quantitative Auswertung erfolgt mittels externem Standard. Analog zur Analyse der DNT-Probe wird eine definierte Menge wässrigen Natriumhydrogencarbonat-Lösung mit einem Gehalt w = 0,5 g NaHCO3/ kg Lösung vorgelegt, mit Schwefelsäure versetzt und gaschromatographisch analysiert.

### Beispiel 1:

Hydrierung von Dinitrotoluol mit einem Gehalt an physikalisch gelöstem oder chemisch gebundenem Kohlendioxid von 0,19 mol-% (nicht erfindungsgemäß)

In einer kontinuierlich betreibbaren, Laborhydrierapparatur, bestehend aus
- einer beheizten, mit Stickstoff überlagerten DNT-Vorlage,
- einer DNT-Dosierpumpe mit beheizten Zu- und Abführleitungen,
- einem 11-Hydrierreaktor, ausgerüstet mit Heiz-/Kühlmantel, innenliegenderKühlschlange und angeschlossenem Heiz-/Kühlsystem, einem Begasungsrührer, abgetauchten Dosierlanzen sowie einer feinporigen Fritte mit angeschlossener Austragsleitung und
- einem beheizten Abscheider zur Phasentrennung mit Druckhaltung, Standmessung und beheizter Austragsleitung zur Produktentnahme
   werden unter Stickstoffbeschleierung 800 g eines Gemisches, enthaltend 62 Gew.-% Toluylendiamin und 38 Gew% Wasser, bezogen auf das Gewicht der Mischung, in den auf 50°C vorgewärmten Reaktor gefüllt und mit einer Suspension von 50 g Wasser und 7 g eines wasserfeuchten Raney-Nickel-Eisen-Katalysa-tors mit einem Eisengehalt von 15 Gew.-%, bezogen auf das Gewicht der Ausgangslegierung, versetzt. Die geschlossene Apparaturwird mit Wasserstoff einer Reinheit von > 99,9 Vol-% auf 30 bar absolut bespannt und der Reaktor unter Inbetriebnahme des Begasungsrührers auf 140 °C aufgeheizt und für2 Stunden zur Katalysatoraktivierung auf Solltemperatur gehalten, dabei die Atmosphäre der Hydrierapparaturim druckführenden Bereich mit einer Purgerate von 30 Normlitern/h von den vorgelegten Inerten befreit.

In die so vorbereitete Apparatur werden 125g/h Dinitrotoluol, das in der Vorlage bei 75°C gelagert ist, mit einem Gehalt an Kohlendioxid in physikalisch gelöster oder chemisch gebundener Form von 0,19 mol-%, dosiert, dabei wird der Druck im System unter Einhaltung der Purgerate von 30 Normlitern/h durch die Einspeisung von Wasserstoff einer Reinheit > 99,9 Vol.% aufrecht erhalten, der Stand im Reaktor über die Fritte auf Sollhöhe gehalten, das ausgetragene, katalysatorfreien Reaktionsgemisch im nach geschalteten Abscheider aufgefangen und diesem periodisch unter Probennahme entnommen.

Die Qualität der Umsetzung wird durch "High-Pressure-Liquid.Chromatographie" verfolgt. Nach einer Laufzeit von 76 h ist kein vollständiger Umsatz mehr zu beobachten, die Chromatogramme zeigen den Durchbruch von Nitro-amino-Aromaten an, der Versuch wird abgebrochen.

### Beispiel 2: Hydrierung von Dinitrotoluol mit einem Gehalt an physikalisch gelöstem oder chemisch gebundenem Kohlendioxid von 0,045 mol-%.

Die Vorbereitung und Durchführung des Versuchs erfolgen analog zu dem Beispiel 1. Zum Einsatz kommt jedoch ein Dinitrotoluol mit einem Gehalt an physikalisch gelöstem oder chemisch gebundenem Kohlendioxid von 0,045 mol-%.

Die Qualität der Umsetzung wird durch "High-Pressure-Liquid.Chromatographie" erfolgt. Erst nach einer Laufzeit von 162 h ist kein vollständiger Umsatz mehr zu beobachten, die Chromatogramme zeigen den Durchbruch von Nitro-amino-Aromaten an, der Versuch wird abgebrochen.

### Beispiel 3: Hydrierung von Dinitrotoluol mit einem Gehalt an physikalisch gelöstem oder chemisch gebundenem Kohlendioxid von 0,18 mol-% (nicht erfindungsgemäß)

Einem mit Stickstoff inertisiertem Rührwerksbehälter werden kontinuierlich 5860 kg/h einer Katalysatorsuspension, enthaltend 704 kg/h (ca. 12 Gew.% bezogen auf das Gewicht der Suspension) eines Raney-Nickel-Eisen-Katalysators mit Eisengehalten von ca. 30 Gew.%, bezogen auf das Gewicht des Katalysators, sowie 27,1 Gew.% m-TDA, 0,9 Gew.% o-TDA, 37 Gew.-% Isopropanol, 23 Gew.% Wasser, jeweils bezogen auf das Gewicht der Suspension, zugeführt und dort kontinuierlich mit 5876 kg eines technischen Dinitrotoluols, enthaltend 99,4 Gew.-% Dinitrotoluol, bezogen auf das Gewichtder Mischung, mit einem Gehalt an Kohlendioxid in physikalisch gelöster oder chemisch gebundener Form von 0,18 mol-%, bezogen auf das eingesetzte Dinitrotoluol sowie 5870 kg aufbereiteten Lösungsmittels, enthaltend 87 Gew.% Isopropanol und 13 Gew.-% Wasser,bezogen auf das Gewicht des aufbereiteten Lösungsmittels, vermischt, dabei beträgt die Betriebstemperatur des Mischbehälters 75 °C.

Die so bereitete Mischung wird kontinuierlich dem Mischbehälterentnommen und einer Hochdruckhydrieranlage zugeführt, dort bei 150 °C und 100 bar in einerbezüglich des umgewälzten Wasserstoffs in Reihe, bezüglich der eingesetzten Dinitrotoluolmischung parallel betriebenen gekühlten Reaktorenkaskade mit Wasserstoff umgesetzt. Das die Reaktorenkaskade mit einer mittleren Verweilzeit von 21 Minuten verlassende Reaktionsgemisch wird auf 75 °C abgekühlt, dann einem Phasenabschelder zugeführt und dort in seine Phasen aufgetrennt.

Die Gasphase wird auf den Anfang der Reaktorenkaskade zurückgeführt, dabei der Wasserstoffgehalt der Gasphase über einen Purgestrom auf Konzentrationen > 90 Vol.-% gehalten. Die Flüssigphase wird dem Abscheider kontinuierlich entnommen, in eine gerührte Filtrationsvorlage entspannt und aus dieser einer Filtratiohseinheit zugeführt. In der Filtrationseinheit wird das Gemisch so aufgetrennt, dass neben einem Klarfiltrat eine Katalysatorsuspension, enthaltend 12 Gew.-% Raney-Nickel-Eisen-Katalysator, 27,1 Gew.-% m-TDA, 0,9 Gew.% o-TDA, 37 Gew.-% Isopropanol und 23 Gew.-% Wasser, erhalten wird. Das abgetrennte Klarfiltrat wird destillativ aufbereitet, die erzeugte Katalysatorsuspension auf den Mischbehälter zurückgeführt.

Die katalytische Aktivität des im Kreis geführten Katalysators wird gaschromatographisch verfolgt, dabei dem Alterungsverhalten des eingesetzten Katalysators dadurch Rechnung getragen, dass stets eine definierte Menge Frischkatalysator in Form einer 1 Gew.-%igen Suspension in Wasser dem Mischbehälter zugeführt sowie über eine periodische Ausschleusung der in der Filtrationseinheit erzeugten Katalysatorsuspension die Sollkonzentration des Katalysatorsin den Reaktionsströmen gehalten wird. Die zur Aufrechterhaltung der katalytischen Aktivität notwendige Einspeisung an Frischkatalysator wird, bezogen auf das hergestellte Toluylendiamin, als spezifischer Katalysatorverbrauch bezeichnet.

Beidem Einsatz von Dinitrotoluol mit einem Gehalt an Kohlendioxid in physikalisch gelöster oder chemisch gebundener Form von 0,18 mol-%, bezogen auf das eingesetzte Dinitrotoluol, wird ein spezifischer Katalysatorverbrauch von 27 g / 100 kg TDA erzielt.

### Beispiel 4: Hydrierung von Dinitrotoluol mit einem Gehalt an physikalisch gelöstem oder chemisch gebundenem Kohlendioxid von 0,045 mol-%

Die Durchführung des Versuchs erfolgt analog zu dem Beispiel 3. Zum Einsatz kommt jedoch ein Dinitrotoluol mit einem Gehalt an physikalisch gelöstem oder chemisch gebundenem Kohlendioxid von 0,045 mol-%.

Bei dem Einsatz von Dinitrotoluol mit einem Gehalt an Kohlendioxid in physikalisch gelöster oder chemisch gebundener Form von 0,046 mol-%, bezogen auf das eingesetzte Dinitrotoluol, wird ein spezifischer Katalysatorverbrauch von 6 g/100 kg TDA erzielt.

## Patentansprüche

1. Verfahren zur Herstellung von Toluylendiamin, bei dem Dinitrotoluol mit Wasserstoff in Gegenwart eines Katalysators umgesetzt wird, **dadurch gekennzeichnet, dass**
a) Toluol mit Nitriersäure zu Mononitrotoluol umgesetzt wird, wobei ein Mononitrotoluole enthaltenes Reaktionsgemisch erhalten wird, und
b) das Mononitrotoluole enthaltende Reaktionsgemisch in eine Mononitrotoluole enthaltende organische Phase und eine Schwefelsäure enthaltende wässrige Phase aufgetrennt wird, und
c) die Mononitrotoluole enthaltende organische Phase mit Nitriersäure umgesetzt wird, wobei ein Dinitrotoluole enthaltendes Reaktionsgemisch erhalten wird, und
d) das Dinitrotoluole enthaltende Reaktionsgemisch in eine Dinitrotoluole enthaltende organische Phase und eine Schwefelsäure enthaltende wässrige Phase aufgetrennt wird, und
e) die Dinitrotoluole enthaltende organische Phase mit Wasser in einer mehrstufigen Extraktion umfassend Vermischung und Phasentrennung in jeder Stufe gereinigt wird, und
f) die so aufbereiteten Dinitrotoluole enthaltend 2,4-DNT in Gehalten von 74- 81 Gew-%, 2,6-DNT in Gehalten von 17 - 21 Gew.-%, sowie 2,3-DNT, 2,5-DNT, 3,4-DNT und 3,5-DNT in Summe in Gehalten < 5,5 Gew.-%, jeweils bezogen auf das Gewicht der eingesetzten Dinitrotoluole, mit Wasserstoff in Gegenwart eines Katalysators zu Toluylendiaminen umgesetzt wird,
wobei
g) die Verweilzeit der Vermischung jeder Stufe der Extraktion in Schritt e) mindestens 4 Minuten und höchstens 60 Minuten, beträgt und
h) zumindest in der letzten Stufe der Extraktion in Schritt e) in die Vermischung zusätzlich ein Inertgas in einem solchen Gewichts-Verhältnis von Inertgas zu den Dinitrotoluolen eingetragen wird, dass die erhaltenen, gereinigten Dinitrotoluole einen Gehalt an Kohlendioxid in physikalisch gelöster oder chemisch gebundener Form in der Summe von weniger als 0,175 mol-%, bezogen auf die molare Menge der Dinitrotoluole, aufweist.

2. Verfahren nach Anspruch 1, **dadurch gekennzeichnet, dass** in Schritt e) zur Extraktion der Dinitrotoluole in mindestens einer Stufe alkalisches Wasser und in mindestens einer Stufe neutrales Wasser eingesetzt wird.

3. Verfahren nach einem der Ansprüche 1 bis 2, dadurch gekenntzeichnet, dass als Katalysatoren Nickel-haltige Katalysatoren eingesetzt werden.

## Claims

1. Process for preparing toluenediamine, in which dinitrotoluene is reacted with hydrogen in the presence of a catalyst, **characterized in that**
a) toluene is reacted with nitrating acid to form mononitrotoluene, giving a reaction mixture containing mononitrotoluenes, and
b) the reaction mixture containing mononitrotoluenes is separated into an organic phase containing mononitrotoluenes and an aqueous phase containing sulphuric acid and
c) the organic phase containing mononitrotoluenes is reacted with nitrating acid to give a reaction mixture containing dinitrotoluenes and
d) the reaction mixture containing dinitrotoluenes is separated into an organic phase containing dinitrotoluenes and an aqueous phase containing sulphuric acid and
e) the organic phase containing dinitrotoluenes is purified by means of water in a multistage extraction comprising mixing and phase separation in each stage and
f) the dinitrotoluenes which have been worked up in this way and contain 2,4-DNT in amounts of 74-81% by weight, 2,6-DNT in amounts of 17-21% by weight and also 2,3-DNT, 2,5-DNT, 3,4-DNT and 3,5-DNT in a total amount of < 5.5% by weight, in each case based on the weight of dinitrotoluenes used, are reacted with hydrogen in the presence of a catalyst to form toluenediamines,
where
g) the residence time for mixing in each stage of the extraction in step e) is at least 4 minutes and not more than 60 minutes and
h) an inert gas is additionally introduced during mixing in at least the last stage of the extraction in step e) in such a weight ratio of inert gas to dinitrotoluenes that the purified dinitrotoluenes obtained have a total content of carbon dioxide in physically dissolved or chemically bound from of less than 0.175 mol%, based on the molar amount of dinitrotoluenes.

2. Process according to Claim 1, **characterized in that** alkaline water is used for the extraction of the dinitrotoluenes in at least one stage in step e) and neutral water is used in at least one stage.

3. Process according to either of Claims 1 and 2, **characterized in that** nickel-containing catalysts are used as catalysts.

## Revendications

1. Procédé de préparation de toluylènediamine, dans lequel du dinitrotoluène est transformé avec de l'hydrogène en présence d'un catalyseur, **caractérisé en ce que**
a) du toluène est transformé avec de l'acide sulfonitrique en mononitrotoluène, avec obtention d'un mélange réactionnel contenant des mononitrotoluènes, et
b) le mélange réactionnel contenant des mononitrotoluènes est séparé en une phase organique contenant des mononitrotoluènes et une phase aqueuse contenant de l'acide sulfurique, et
c) la phase organique contenant des mononitrotoluènes est transformée avec de l'acide sulfonitrique, avec obtention d'un mélange réactionnel contenant des dinitrotoluènes, et
d) le mélange réactionnel contenant des dinitrotoluènes est séparé en une phase organique contenant des dinitrotoluènes et une phase aqueuse contenant de l'acide sulfurique, et
e) la phase organique contenant des dinitrotoluènes est purifiée avec de l'eau dans une extraction à plusieurs étapes, comprenant un mélange et une séparation de phases dans chaque étape, et
f) les dinitrotoluènes ainsi préparés, contenant du 2,4-DNT en des teneurs de 74-81% en poids, du 2,6-DNT en des teneurs de 17-21% en poids, ainsi que du 2,3-DNT, du 2,5-DNT, du 3,4-DNT et du 3,5-DNT au total en des teneurs < 5,5% en poids, à chaque fois par rapport au poids des dinitrotoluènes utilisés, sont transformés avec de l'hydrogène en présence d'un catalyseur en toluylènediamines,
où
g) le temps de séjour du mélange dans chaque étape de l'extraction dans l'étape e) est d'au moins 4 minutes et d'au plus 60 minutes et
h) on introduit au moins dans la dernière étape de l'extraction dans l'étape e) dans le mélange, en plus, un gaz inerte dans un rapport pondéral de gaz inerte aux dinitrotoluènes tel que les dinitrotoluènes purifiés, obtenus présentent une teneur en dioxyde de carbone sous forme physiquement dissoute ou chimiquement liée au total inférieure à 0,175% en mole, par rapport à la quantité molaire des dinitrotoluènes.

2. Procédé selon la revendication 1, **caractérisé en ce qu'**on utilise dans l'étape e), pour l'extraction des dinitrotoluènes, dans au moins une étape de l'eau alcaline et dans au moins une étape de l'eau neutre.

3. Procédé selon l'une quelconque des revendications 1 à 2, **caractérisé en ce qu'**on utilise comme catalyseurs des catalyseurs contenant du nickel.
